(19) Europäisches Patentamt
European Patent Office
Office européen des brevets

(11) Veröffentlichungsnummer: **0 291 872 B1**

(12) # EUROPÄISCHE PATENTSCHRIFT

(45) Veröffentlichungstag der Patentschrift: **26.08.92**

(51) Int. Cl.5: **C07C 69/26**, C07C 69/28, C07C 69/30, C07C 67/26, C07C 67/08, B29K 91/00

(21) Anmeldenummer: **88107705.1**

(22) Anmeldetag: **13.05.88**

(54) **Wachsartige Verbindungen aromatischer Alkohole und ihre Verwendung.**

(30) Priorität: **19.05.87 DE 3716723**

(43) Veröffentlichungstag der Anmeldung:
**23.11.88 Patentblatt 88/47**

(45) Bekanntmachung des Hinweises auf die Patenterteilung:
**26.08.92 Patentblatt 92/35**

(84) Benannte Vertragsstaaten:
**BE DE ES FR GB IT NL**

(56) Entgegenhaltungen:
**EP-A- 0 011 325**
**US-A- 3 132 164**

**ZEITSCHRIFT FUER NATURFORSCHUNG, Sektion C, Band 41c, 1986, Seiten 673-676, Tübingen; P. G. GUELZ et al, "Esters of Benzyl Alcohol and 2-phenyl-ethanol-1 in Epicuticular Waxes from Jojoba Leaves"**

(73) Patentinhaber: **HOECHST AKTIENGESELLSCHAFT**
**Postfach 80 03 20**
**W-6230 Frankfurt am Main 80(DE)**

(72) Erfinder: **Maier, Karl**
**Dresdener Strasse 51**
**W-8900 Augsburg(DE)**
Erfinder: **Hohner, Gerd, Dr.**
**Adolf-von-Baeyer-Strasse**
**W-8906 Gersthofen(DE)**
Erfinder: **Piesold, Jan-Peter, Dr.**
**Oblatterwallstrasse 44**
**W-8900 Augsburg(DE)**

EP 0 291 872 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf die Verwendung Wachsester aromatischer Alkohole als Gleit- und Trennmittel für thermoplastische Kunststoffe.

Der Einsatz von Gleit- und Trennmitteln bei der Verarbeitung von Polyvinylchlorid, Polystyrol, Polyestern und anderen Thermoplasten, ist schon lange bekannt und allgemein gebräuchlich. Als solche werden bevorzugt Fettsäurederivate, langkettige Alkohole sowie Montanwachse eingesetzt. Die Fettsäurederivate, wie beispielsweise das N,N'-Ethylen-bis-stearamid, verbessern zwar das Fließverhalten der Polymerschmelze und ergeben hochtransparente Endprodukte, störend ist jedoch ihre Flüchtigkeit und Geruchsentwicklung bei den häufig auftretenden hohen Verarbeitungstemperaturen. Die bisher gebräuchlichen Montanwachse zeigen zwar nicht diese störenden Eigenschaften, ihre Zugabemenge ist jedoch begrenzt, da sie Trübungen verursachen können.

Bekannt sind wachsartige Ester von Phenolen, aromatischen, aliphatischen oder cycloaliphatischen Alkoholen mit langkettigen Carbonsäuren, welche als Gleitmittel bei der PVC-Verarbeitung dienen sollen (vgl. EP-PS 11325). Allerdings wird in dieser Druckschrift eine Vielzahl von Phenolen und Alkoholen genannt, und die Carbonsäuren sollen zu mindestens 40 Gew.-% verzweigtkettig sein.

Weiterhin ist die Verwendung einer Verbindung der Formel

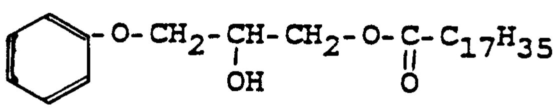

als Gleitmittel für die Verarbeitung von PVC beschrieben worden (vgl. JP-OS 76.147 545). Die Verbindung soll die Verarbeitbarkeit des PVC verbessern. Ob dies auch für die Transparenz des Formteils gilt, ist nicht offenbart.

Bekannt sind Benzyltetracosanoat, Benzylhexacosanoat, Benzyloctacosanoat und Benzyltriacontanoat als Bestandteile in Wachsüberzügen auf Jojoba - Blättern (vgl. Z. Naturforsch. 41c, 673-676 (1986)).

Es bestand somit die Aufgabe, Gleit- und Trennmittel zu finden, welche die positiven Eigenschaften der Montanwachse ohne deren Neigung zu Trübungen besitzen.

Die Erfindung betrifft daher die Verwendung der in den Ansprüchen beschriebenen wachsartigen Verbindungen als Gleit- und Trennmittel für thermoplastische Kunststoffe.

Die erfindungsgemäß zu verwendenden Wachsester sind Verbindungen der Formel (I)

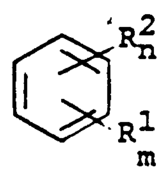

(I),

sie entstehen durch Umsetzung von Verbindungen der Formel (II)

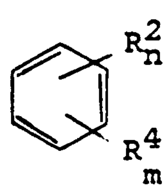

(II)

oder der Formel (IV)

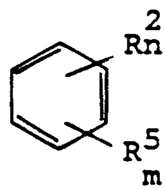

(IV)

mit Carbonsäuren der Formel (III)

$R^3$-COOH (III) .

In diesen Formeln bedeuten

$R^1$ die Gruppe

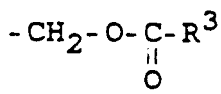

oder

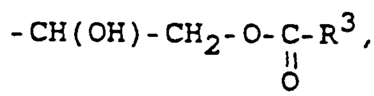

$R^2$ einen Alkylrest mit 1 bis 4 C-Atomen oder Halogen, vorzugsweise einen Alkylrest mit 1 bis 2 C-Atomen, oder Chlor,

$R^3$ einen unverzweigten Alkylrest mit 20 bis 36 C-Atomen, vorzugsweise mit 26 bis 32 C-Atomen,

$R^4$ die Gruppe $-CH_2OH$ oder $-CH(OH)-CH_2OH$, vorzugsweise $-CH_2-OH$,

$R^5$ die Gruppe

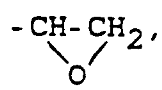

m 1, 2 oder 3, vorzugsweise 1 oder 2, und

n null bis (6-m), vorzugsweise null bis 3.

Geeignete Ausgangsverbindungen der Formel (II) sind beispielsweise Benzylalkohol, o-, m-, p-Tolylcarbinol, Chlorbenzylalkohol, Brombenzylalkohol, 2,4-Dimethylbenzylalkohol, 3,5-Dimethylbenzylalkohol,2,3,5-Cumobenzylalkohol, 3,4,5-Trimethylbenzylalkohol, p-Cuminalkohol, 1,2-Phthalylalkohol, 1,3-Bis(hydroxymethyl)benzol (= m-Xylylenalkohol), 1,4-Bis(hydroxymethyl)benzol (= p-Xylylenalkohol), Pseudocumenylglykol, Mesitylenglykol, Mesitylenglycerin.

Eine geeignete Ausgangsverbindung der Formel (IV) ist beispielsweise Styroloxyd.

Geeignete Carbonsäuren der Formel (III) sind beispielsweise Arachinsäure, Behensäure, Tetracosansäure, Cerotinsäure, Montansäure, Melissinsäure, vorzugsweise Cerotinsäure, Montansäure und Melissinsäure, insbesondere technische Montansäure, welche im wesentlichen ein Gemisch aus $C_{18}$-$C_{36}$-Carbonsäuren mit einem überwiegenden Anteil an $C_{26}$-$C_{32}$-Carbonsäuren ist und durch oxidative Bleichung von Rohmontanwachs gewonnen wird.

Die Umsetzung des Alkohols der Formel (II) mit der Carbonsäure der Formel (III) kann in einem inerten Lösemittel stattfinden. Geeignete Lösemittel sind aliphatische und aromatische Kohlenwasserstoffe, vorzugsweise aromatische Kohlenwasserstoffe wie beispielsweise Toluol oder Xylol.

Vorzugsweise wird die Umsetzung jedoch in Abwesenheit eines Lösemittels durchgeführt, indem die Carbonsäure geschmolzen vorgelegt wird und der Alkohol in die Schmelz eingetragen wird. Das entstehende Reaktionswasser wird abdestilliert. Der Fortgang der Reaktion wird durch laufende Bestimmung der

Säurezahl überwacht. Die Reaktion findet in Gegenwart eines Katalysators statt, wie beispielsweise Schwefelsäure, Toluol- oder Naphthalinsulfonsäure oder Zinn- oder Zinksalze. Vorzugsweise wird Schwefelsäure verwendet. Alkohol und Carbonsäure werden im Molverhältnis 2,0:1 bis 0,1:1, vorzugsweise 1,0:1 bis 0,5:1 eingesetzt. Die Reaktionstemperatur liegt im Bereich von 50 bis 200 °C, vorzugsweise 90 bis 160 °C. Die Carbonsäure kann sowohl vollständig als auch teilweise bis zu einer gewünschten Restsäurezahl des Gemisches verestert werden. Im Überschuß vorhandener Alkohol kann nach der Reaktion durch Abdestillieren, Extrahieren oder ein ähnliches Verfahren abgetrennt werden.

Die Herstellung der Verbindungen der Formel (I) mit

$$R^1 = -CH(OH)-CH_2-O-\underset{\underset{O}{\|}}{C}-R^3$$

kann auch in der Weise erfolgen, daß ein Epoxid der Formel (IV) umgesetzt wird mit einer Carbonsäure der Formel (III), wobei ein Katalysator zugegen ist, beispielsweise Chrom(III)-Salze, aliphatische oder aromatische Amine, Aluminiumoxid, vorzugsweise aliphatische tertiäre Amine wie Trimethyl- oder Triethylamin. Im übrigen wird analog verfahren wie bei der Reaktion zwischen Alkohol und Carbonsäure.

Nach ihrer Bildung können die erfindungsgemäß zu verwendenden Ester mit Alkali- oder Erdalkalibasen behandelt werden, um überschüssige Carbonsäure zu neutralisieren oder durch partielle Verseifung einen bestimmten Anteil Metallseifen zu bilden. Für die partielle Verseifung wird bevorzugt Calciumhydroxid eingesetzt.

Die Verbindungen der Formel (I) sind weiße bis gelbliche Wachse mit einer Säurezahl im Bereich von 0 bis 190, einer Verseifungszahl im Bereich von 90 bis 200 und einem Tropfpunkt von 60 bis 120 °C.

Sie besitzen eine gleichbleibend gute Gleit- und Trennwirkung, verglichen mit den bisher gebräuchlichen Montanwachsestern aliphatischer Alkohole, bewirken jedoch eine deutlich bessere Transparenz. Aufgrund des sehr guten Transparenzverhaltens werden die erfindungsgemäß zu verwendenden Wachsester bevorzugt bei der Verarbeitung von hochtransparenten thermoplastischen Kunststoffen eingesetzt.

Solche hochtransparenten thermoplastischen Kunststoffe werden erhalten beispielsweise durch die Polymerisation von Vinylchlorid, Vinylidenchlorid, Ethylvinylacetat, Acrylnitril, Acrylsäure(estern), Methacrylsäure(estern), Styrol, $\alpha$-Methylstyrol, p-Methylstyrol oder Mischungen dieser Monomeren. Andere hochtransparente thermoplastische Kunststoffe, für deren Verarbeitung sich die Verwendung der Esterwachse besonders anbietet, sind Polykondensationsprodukte, wie z.B. Polycarbonat, Polyethylenterephthalat, Polybutylenterephthalat, Polyphenylenoxid, Polysulfone, Polyethersulfone und Polyarylether.

Weiterhin können die Wachsester auch bei der Verarbeitung von Polymermischungen, sogenannten Polymerblends, angewendet werden.

Obgleich der besondere Vorteil der erfindungsgemäß zu verwendenden Esterwachse auf dem Gebiet der hochtransparenten thermoplastischen Formmassen liegt, sind sie selbstverständlich auch bei nichttransparenten Formmassen einsetzbar.

Der Anteil des Wachses an der Formmasse beträgt 0,01 bis 5 Gew.-%, vorzugsweise 0,05 bis 2,5 Gew.-%, insbesondere 0,5 bis 1 Gew.-%.

Zusätzlich können die Formmassen noch weitere Additive enthalten, wie beispielsweise Antioxidantien, beispielsweise alkylierte Monophenole, alkylierte Hydrochinone, hydroxylierte Thiodiphenylether, Alkyliden-Bisphenole, Benzylverbindungen, Acylaminophenole, Ester der $\beta$-(5-tert. butyl-4-hydroxy-3-methylphenyl)-propionsäure, Amide der $\beta$-(3,5-Di-tert. butyl-4-hydroxyphenyl)-propionsäure. UV-Absorber und Lichtschutzmittel, beispielsweise 2-(2'-Hydroxyphenyl)-benztriazole, 2-Hydroxybenzophenone, Ester von gegebenenfalls substituierten Benzoesäuren, Acrylate, Nickelverbindungen, sterisch gehinderte Amine, Oxalsäurediamide, Metalldesaktivatoren, Phosphate und Phosphonite, peroxidzerstörende Verbindungen, basische Costabilisatoren, Nukleierungsmittel, Füllstoffe und Verstärkungsmittel, Weichmacher, optische Aufheller, Flammschutzmittel, Antistatika, Treibmittel und andere Gleitmittel.

Das Einarbeiten der Wachsester in die Formmassen geschieht auf bekannte Weise, beispielsweise mittels Mischer, Extruder, Walzwerken oder Kneten.

Die folgenden Beispiele sollen die Erfindung erläutern. Die angegebenen Mengen beziehen sich auf das Gewicht.

**Beispiel 1**

500 g technische Montanwachssäure (SZ 138, 1,23 mol) wurden in einem Wittschen Topf (mit Rührer,

Thermometer, Destillationsbrücke, $N_2$-Überlagerung) aufgeschmolzen und bei 120 °C mit 131,6 g (1,22 mol) Benzylalkohol sowie 0,5 ml $H_2SO_4$ (44 %ig) versetzt. Man rührte solange, bis die Säurezahl auf ca. 25 mg KOH/g gesunken war (ca. 3 h), wobei ein Gemisch aus Reaktionswasser und Benzylalkohol überdestillierte. Anschließend wurde der Ansatz mit wäßriger Natronlauge gegen Thymolblau neutralisiert, mit 5 ml $H_2O_2$ (30 %ig) gebleicht, unter vermindertem Druck von flüchtigen anteilen befreit und druckfiltriert. Man erhielt ein helles Wachs.

| | |
|---|---|
| Säurezahl | 26 |
| Verseifungszahl | 159 |
| Tropfpunkt | 76 °C |

**Beispiel 2**

100 g technische Montanwachssäure (SZ 110, 0,20 mol) wurden in einem 250 ml-Dreihalskolben (mit Rührer, Thermometer, Tropftrichter) aufgeschmolzen. Bei 140 °C Innentemperatur wurden 0,2 g Triethylamin zugegeben und anschließend innerhalb von 30 min 23,2 g (0,19 mol) Styroloxid zugetropft. Nach ca. 2 h blieb die Säurezahl konstant bei 18. Flüchtige Anteile wurden durch Anlegen von vermindertem Druck entfernt. Man erhielt ein helles Wachs.

| | |
|---|---|
| Säurezahl | 17 |
| Verseifungszahl | 124 |
| Tropfpunkt | 81 °C |

In den folgenden Beispielen bedeuten

Wachs 1: Montansäureester des Ethylenglykols,
Wachs 2: Montansäureester des 1,3-Butandiols, teilverseift (1,4 Gew.-% Ca),
Wachs 3: Montansäureester des Glycerins,
Wachs 4: Montansäureester des Benzylalkohols,
Wachs 5: Montansäureester des Benzylalkohols, teilverseift (1,4 Gew.-% Ca),
Wachs 6: Montansäureester des $\beta$-Hydroxy-$\beta$-phenylethanols, teilverseift (1,4 Gew.% Ca),
Wachs 7: Montansäureester des 1,4-Bis(hydroxymethyl)benzols, teilverseift (1,4 Gew.-% Ca),

Die Wachse 1, 2 und 3 sind handelsüblich und dienen als Vergleich, die Wachse 4 bis 7 sind erfindungsgemäß.

**Beispiel 3**

100,0 T. S-PVC (K-Wert 60)
1,0 T. Verarbeitungshilfsmittel auf PMMA-Basis
1,5 T. Dioctylzinnthioglycolat
0,3 T. Glycerinmonostearat und
0,6 T. Prüfprodukt

wurden auf einem Prüfwalzwerk bei einer Temperatur von 190 °C plastiziert und anschließend zu 0,5 bzw. 2,0 mm dicken Platten verpreßt. Diese wurden auf ihre Transparenz geprüft.

| Wachs | Transparenz/0,5 mm | Transparenz/2,0 mm |
|---|---|---|
| 1 | 68,2 | 41,4 |
| 2 | 83,4 | 71,6 |
| 3 | 77,1 | 54,4 |
| 5 | 88,8 | 84,4 |
| 6 | 87,1 | 82,5 |
| 7 | 84,6 | 71,0 |

**Beispiel 4**

Wie Beispiel 1 mit 5,0 T. Schlagzähmacher
Die Gleitmitteleignung wurde in einem Brabender-Kneter (32 g Einwaage; 40 Upm; 160 °C; 2 kg Belastung) und auf einem Prüfwalzwerk (190 °C; 15/20 Upm) getestet.

| | Kneter | | | Walzwerk | |
|---|---|---|---|---|---|
| Wachs | Plastizierzeit min | maximales Drehmoment Nm | konstantes Drehmoment Nm | klebfreie Zeit min | Endstabilität |
| 1 | 1,8 | 36,0 | 23,8 | 21 | 40 |
| 2 | 1,8 | 33,0 | 23,2 | 30 | 40 |
| 5 | 1,2 | 37,0 | 24,0 | 21 | 40 |
| 6 | 1,3 | 36,1 | 23,7 | 27 | 43 |
| 7 | 1,0 | 35,5 | 22,8 | 27 | 39 |

**Beispiel 5**

Ein Polyethylenterephthalat mit der spezifischen Viskosität von 1060 (SV-Wert) wurde mit 0,3 Gew.-% verschiedener Wachse vermischt. Aus dieser Mischung wurden auf einer Spritzgußmaschine 1 mm-Plättchen gespritzt (Massetemperatur 280 °C, Werkzeugtemperatur 30 °C).

| Wachs | Transparenz |
|---|---|
| ohne | 86,6 |
| 1 | 79,0 |
| 2 | 78,2 |
| 4 | 85,0 |

**Beispiel 6**

Polystyrol (MFI 200/5: 25 g/10 min; DIN 53735) wurde bei 180 °C 10 Minuten im Brabender-Kneter mit jeweils 1 % Wachs gemischt. Danach wurde die Schmelze zu 2 mm dicken Platten verpreßt, die auf ihre

Transparenz untersucht wurden.

| Wachs | Knetwiderstand | Transparenz | Anmerkung |
|---|---|---|---|
| ohne | 8,8 Nm | 83,7 % | klebt im Brabender |
| 1 | 7,8 Nm | 53,7 % | Überschmierung |
| 4 | 8,5 Nm | 83,8 % | kein Kleben |
| 6 | 8,5 Nm | 82,4 % | kein Kleben |
| 7 | | 64,5 % | kein Kleben |

HKV-Messungen bestätigten die guten Gleitmitteleigenschaften der neuen Montanwachse. Hierfür wurden die oben beschriebenen Platten granuliert, die Messungen erfolgten bei 170 °C mit einer 1 mm-Düse (L/D = 30).

| Wachs | Ausstoß in g/10 min | | | |
|---|---|---|---|---|
| 1 % | 115 | 170 | 225 | 280 |
| ohne | 3,1 | 8,7 | 20,2 | 38,8 |
| 1 | 4,0 | 11,3 | 28,6 | 52,7 |
| 4 | 3,9 | 11,2 | 27,7 | 49,9 |
| 6 | 4,0 | 12,3 | 30,3 | 51,2 |
| 7 | 3,6 | 10,4 | 25,1 | - |

## Patentansprüche

1. Verwendung einer wachsartigen Verbindung der Formel (I),

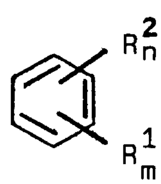

(I),

worin $R^1$ die Gruppe

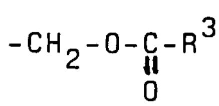

oder

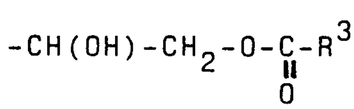

,

und $R^2$ ein Alkylrest mit 1-4 C-Atomen oder Halogen,
$R^3$ ein unverzweigter Alkylrest mit 20-36 C-Atomen,

m 1,2 oder 3 und n 0 bis (6-m) ist, als Gleit- und Formtrennmittel für thermoplastische Formmassen.

**2.** Formmasse, im wesentlichen bestehend aus einem thermoplastisch verarbeitbarem Polymeren und 0,01-5,00 %, bezogen auf die Formmasse, einer Verbindung gemäß Anspruch 1.

**3.** Thermoplastische Formmasse gemäß Anspruch 2, dadurch gekennzeichnet, daß das Polymere ein Polyvinylchlorid, Polyvinylidenchlorid, Polystyrol, Poly-α-methylstyrol, Poly-p-methylstyrol, Poly(meth)-acrylat, Polyether, Polycarbonat, Polyamid, Polyetherketon, Polysulfon, Polyethersulfon oder eine Mischung dieser Polymeren ist.

## Claims

**1.** Use of a waxy compound of the formula (I)

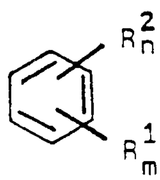

(I)

in which $R^1$ is the group

$$-CH_2-O-\underset{\overset{\|}{O}}{C}-R^3$$

or

$$-CH(OH)-CH_2-O-\underset{\overset{\|}{O}}{C}-R^3,$$

and $R^2$ is an alkyl radical with 1-4 carbon atoms or halogen,
$R^3$ is an unbranched alkyl radical with 20-36 carbon atoms,
m is 1, 2 or 3 and n is 0 to (6-m), as a lubricant or mold release agent for thermoplastic molding materials.

**2.** A molding material essentially consisting of a polymer which can be processed as a thermoplastic and 0.01-5.00%, based on the molding material, of a compound as claimed in claim 1.

**3.** A thermoplastic molding material as claimed in claim 2, in which the polymer is a polyvinyl chloride, polyvinylidene chloride, polystyrene, poly-α-methylstyrene, poly-p-methylstyrene, poly-(meth)acrylate, polyether, polycarbonate, polyamide, polyether-ketone, polysulfone or polyether-sulfone or a mixture of these polymers.

## Revendications

**1.** Utilisation d'un composé cireux de formule (I)

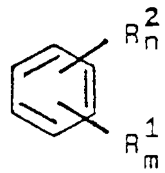

(I),

dans laquelle $R^1$ représente le groupe

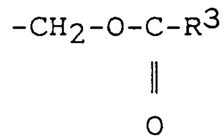

ou

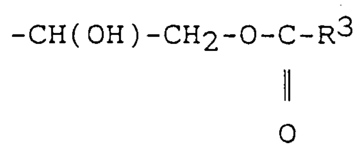

et $R^2$ est un radical alkyle ayant 1 à 4 atomes de carbone ou un halogène, $R^3$ est un radical alkyle non ramifié ayant 20 à 36 atomes de carbone, m vaut 1, 2 ou 3 et n vaut de 0 à (6-m) en tant que lubrifiant et agent de démoulage pour mélanges à mouler thermoplastiques.

**2.** Mélange à mouler, constitué essentiellement d'un polymère pouvant subir une mise en oeuvre thermoplastique et de 0,01-5,00 %, par rapport au mélange à mouler, d'un composé selon la revendication 1.

**3.** Mélange à mouler thermoplastique selon la revendication 2, caractérisé en ce que le polymère est un poly(chlorure de vinyle), un poly(chlorure de vinylidène), un polystyrène, un poly($\alpha$-méthylstyrène), un poly(p-méthylstyrène), un poly(méth)acrylate, un polyéther, un polycarbonate, un polyamide, une polyéthercétone, une polysulfone, une polyéthersulfone ou un mélange de ces polymères.